(19) **Europäisches Patentamt / European Patent Office / Office européen des brevets**

(11) **EP 3 626 167 B1**

(12) ## EUROPEAN PATENT SPECIFICATION

(45) Date of publication and mention
of the grant of the patent:
**17.07.2024 Bulletin 2024/29**

(21) Application number: **18195846.3**

(22) Date of filing: **21.09.2018**

(51) International Patent Classification (IPC):
**A61B 5/024** $^{(2006.01)}$ **A61B 5/00** $^{(2006.01)}$

(52) Cooperative Patent Classification (CPC):
**A61B 5/02416; A61B 5/7267; G16H 50/70**

(54) **A METHOD OF GENERATING A MODEL FOR HEART RATE ESTIMATION FROM A PHOTOPLETHYSMOGRAPHY SIGNAL AND A METHOD AND A DEVICE FOR HEART RATE ESTIMATION**

VERFAHREN ZUM ERZEUGEN EINES MODELLS ZUR HERZFREQUENZSCHÄTZUNG AUS EINEM PHOTOPLETHYSMOGRAPHIE-SIGNAL UND EIN VERFAHREN UND EINE VORRICHTUNG ZUR HERZFREQUENZSCHÄTZUNG

PROCÉDÉ DE GÉNÉRATION D'UN MODÈLE D'ESTIMATION DE DÉBIT CARDIAQUE À PARTIR D'UN SIGNAL DE PHOTOPLÉTHYSMOGRAPHIE AINSI QU'UN PROCÉDÉ ET UN DISPOSITIF D'ESTIMATION DE DÉBIT CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.03.2020 Bulletin 2020/13**

(73) Proprietors:
- **IMEC vzw**
  **3001 Leuven (BE)**
- **Stichting IMEC Nederland**
  **5656 AE Eindhoven (NL)**
- **Regents Of The University Of Minnesota**
  **Minneapolis, Minnesota 55455 (US)**

(72) Inventors:
- **Biswas, Dwaipayan**
  **3001 Leuven (BE)**
- **Everson, Luke**
  **3001 Leuven (BE)**
- **Konijnenburg, Mario**
  **3001 Leuven (BE)**
- **Van Hoof, Christiaan**
  **3001 Leuven (BE)**
- **Van Helleputte, Nick**
  **3001 Leuven (BE)**

(74) Representative: **AWA Sweden AB**
**Box 5117**
**200 71 Malmö (SE)**

(56) References cited:
**WO-A1-2018/078213      US-A1- 2015 282 724
US-A1- 2018 249 964**

- **MOYA-ALBOR ERNESTO ET AL: "Heart Rate Estimation using Hermite Transform Video Magnification and Deep Learning*", 2018 40TH ANNUAL INTERNATIONAL CONFERENCE OF THE IEEE ENGINEERING IN MEDICINE AND BIOLOGY SOCIETY (EMBC), IEEE, 18 July 2018 (2018-07-18), pages 2595 - 2598, XP033428928, DOI: 10.1109/EMBC.2018.8512879**
- **VASU JINDAL: "Integrating Mobile and Cloud for PPG Signal Selection to Monitor Heart Rate During Intensive Physical Exercise", 2016 IEEE/ACM INTERNATIONAL CONFERENCE ON MOBILE SOFTWARE ENGINEERING AND SYSTEMS, 16 May 2016 (2016-05-16), pages 36 - 37, XP033050476**
- **EVERSON LUKE ET AL: "BiometricNet: Deep Learning based Biometric Identification using Wrist-Worn PPG", 2018 IEEE INTERNATIONAL SYMPOSIUM ON CIRCUITS AND SYSTEMS (ISCAS), IEEE, 27 May 2018 (2018-05-27), pages 1 - 5, XP033434430, DOI: 10.1109/ISCAS.2018.8350983**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- YANG WEN ET AL: "Heart Rate Estimation from Facial Videos Based on Convolutional Neural Network", 2018 INTERNATIONAL CONFERENCE ON NETWORK INFRASTRUCTURE AND DIGITAL CONTENT (IC-NIDC), IEEE, 22 August 2018 (2018-08-22), pages 45 - 49, XP033441727, ISBN: 978-1-5386-6066-9, [retrieved on 20181106], DOI: 10.1109/ICNIDC.2018.8525602
- "Deep Learning", 1 January 2016, MIT PRESS, article GOODFELLOW IAN ET AL: "Chapter 1 - Introduction", pages: 1 - 26, XP055799378

## Description

Technical field

The present inventive concept relates to estimating a heart rate based on a photoplethysmography (PPG) signal. In particular, the present inventive concept relates to generating a model for heart rate estimation from a PPG signal and using the model for heart rate estimation.

Background

Acquisition of signals representing a heart activity of a subject is important or of interest in many contexts. Heart rate estimation may be of particular interest. The estimated heart rate may be used in clinical settings to provide information for treatment of the subject, but may also be used for general monitoring of a physical condition of the subject. Also, monitoring of heart activity may be of interest to the subject, e.g. for monitoring exercise or other activities of the subject.

Photoplethysmography (PPG) is a technology of interest in monitoring heart activity as it may be provided in a wearable device which may minimally affect a comfort of the subject wearing the device. For instance, a PPG signal may be acquired from peripheral positions on the subject, such as earlobes, fingertips or wrist. Thus, using sensor for acquiring a PPG signal may be advantageous over sensors acquiring an electrocardiography (ECG) signal, as ECG sensor may require placement of ground and reference sensors proximal to the chest of the subject. PPG sensors may provide a small form factor and may be embedded into a wearable device, which may provide heart rate estimation for unobtrusive daily usage. In particular, the PPG sensor may be embedded into a wristband.

However, the PPG signal may be affected by motion artifacts. Thus, if the subject is moving, motion artifacts may be generated which may cause quality of an acquired PPG signal to deteriorate or may prevent making any assessments based on the PPG signal. This is accentuated, when the PPG signal is acquired during daily life of a subject. For instance, motion artifacts may be caused by physical activity of the subject, by ambient light leaking through a widening gap between sensor and a skin surface of the subject during motion and a change in blood flow due to movements. The motion artifacts may cause a spectral component of the artifacts to overpower a heart-beat related PPG component, such that the motion artifact may prevent accurate estimation of the heart rate.

Attempts have been made to enable robust heart rate estimation, even when motion artifacts are present in the signal. In Vasu Jindal, "Integrating Mobile and Cloud for PPG Signal Selection to Monitor Heart Rate During Intensive Physical Exercise", 2016 IEEE/ACM International Conference on Mobile Software Engineering and Systems, May 16-17, 2016, page 36-37, a technique is disclosed for accurately determining heart rate during intensive motion by classifying PPG signals obtained from smartphones or wearable devices combined with motion data obtained from accelerometer sensors. 17 different features are extracted from each segment of the PPG signal. These features are used for unsupervised learning of Restricted Boltzmann Machines (RBMs) as well as supervised learning of deep belief network (DBN) models. The mobile framework self-learns with each future incoming PPG segment and tags each segment with the associated motion of the source identified using the accelerometer. If the PPG signal quality is classified within a reasonable threshold by the trained DBN then the observation is used to calculate heart rate.

However, the selection of hand-picked/crafted features to be extracted from the segments of the PPG signal may introduce bias into the model and involves heuristic knowledge. Thus, the heart rate estimation from PPG signals may be improved to provide a more robust heart rate estimation even in presence of motion artifacts.

Moya-Albor et al: "Heart Rate Estimation using Hermite Transform Video Magnification and Deep Learning", 2018 40th Annual International Conference of the IEEE Engineering in Medicine and Biology Society (EMBC), 18 July 2018, pages 2595-2598 discloses monitoring of heart rate without contact using video magnification for detection of a pulse signal and a deep learning technique to estimate the beat by beat pulse signal.

US 2018/0249964 A1 discloses that a system that detects heartbeats includes a sensor or a transducer and algorithms based on deep learning. The algorithms employ techniques of artificial intelligence that enable the system to extract heartbeat features under low signal-to-noise-ratio (SNR) conditions when a user is exercising. The algorithms can be applied to various technologies for heart rate monitoring such as ultrasound Doppler, photoplethysmogram (PPG), electrocardiogram (EKG), acoustic, pressure/force sensing and laser/RF Doppler, among other types of sensing methods.

Summary

An objective of the present inventive concept is to enable improved heart rate estimation from PPG signals in presence of motion artifacts. Another objective of the present inventive concept is to generate a model for heart rate estimation from a PPG signal, such that the PPG signal may be used without need of any additional data to estimate heart rate of a subject.

These and other objectives of the invention are at least partly met by the invention as defined in the independent claims. Preferred embodiments are set out in the dependent claims.

According to a first aspect, there is provided a computer-implemented method of generating a model for heart rate estimation from a photoplethysmography (PPG) signal of a subject, said method comprising: receiving subject-specific training data for machine learning, said training data comprising a PPG signal acquired by a wearable device from the subject and a heart rate indicating signal from the subject, wherein the heart rate indicating signal provides a ground truth of heart rates of the subject for associating a heart rate with a time period of the PPG signal; using associated pairs of a heart rate and a complete dataset of a time-series of a PPG signal over a time period as input to a deep neural network (DNN); and determining, through the DNN, a subject-specific model relating the PPG signal of the subject to the heart rate of the subject.

According to the inventive concept, a DNN is taught based on a complete PPG signal. Thus, the model may be determined without a need for prior extraction features from the PPG signal.

The training data is subject-specific, which implies that the model is created to relate PPG signals of a specific subject to heart rate. Thus, the DNN may be taught to provide a model which may enable correct heart rate estimation in relation to typical artifacts of the subject, such as motion artifacts or other variations in the heart signal of the subject. The physiology is different between different subjects which also implies that variations in PPG signals and artifacts occur between different subjects. However, since a subject-specific model is formed, the variation in artifacts may be relatively limited to allow the DNN to correctly model the PPG signals to heart rates while allowing the complete signal to be used as input to the DNN.

Thanks to the invention, a model may be generated, which learns the underlying subject-specific data distribution. This enables an accurate model to be formed so as to allow reliable heart rate estimation based on the PPG signal.

In generating the model, a heart rate indicating signal is included in the training data. The heart rate indicating signal may thus be related to the PPG signal so that the DNN knows the true heart rate, which should be extracted from the PPG signal once the model has been generated. The model is thus formed to relate the PPG signal to heart rates of the subject. Once the model has been generated and is deployed, heart rate estimation is enabled based on only the PPG signal and there is no need of any other additional signal in order to estimate the heart rate of the subject.

The heart rate indicating signal used in the training data may be any signal which provides a reliable heart rate of the subject. Thus, the heart rate indicating signal may for instance be an electrocardiography (ECG) signal, which may be acquired by electrodes worn e.g. on the chest of the subject. The ECG signal could be acquired by a strap carrying electrodes and being worn around the chest of the subject. However, it should be realized that any other reliable heart rate indicating signal may be used, such as using sound sensors or accelerometers to determine heart sound or movements associated with the heart activity.

It should be realized that the DNN need not necessarily generate the subject-specific model from scratch. Rather, a general model for relating a PPG signal to heart rate may be stored based on previously determined models. The general model may be associated with certain characteristics of the subject, such as heart condition, age, gender, body mass index, etc. Thus, the DNN may use the general model as input to adapt the general model to the subject-specific training data for forming the subject-specific model.

However, as an alternative, the DNN does not use any predetermined information on relations between PPG signals and heart rates. Rather, the DNN may form the subject-specific model based merely on the information in the subject-specific training data.

As used herein, the phrase "a complete dataset of a time-series of a PPG signal" should be understood as an entire PPG signal within a time period. Thus, specific features of the PPG signal are not extracted, before the PPG signal is provided as input to the DNN. Rather, the DNN may receive a raw PPG signal, which is raw in terms of specific features not being extracted from the PPG signal. However, it should be realized that some pre-processing of the PPG signal may be performed, which does not correspond to extracting features. For instance, the PPG signal may be subject to filtering, such as bandpass filtering, in order to remove clearly irrelevant frequencies of the PPG signal, which may not indicate the heart rate. Thus, the PPG signal may be pre-processed such that frequencies which cannot physically correspond to a heart rate may be removed. Such pre-processing still implies that information of the PPG signal in each time instance is present in the signal so as to form a complete dataset of a time-series. Further, it should be realized that the PPG signal may be formed into a digital signal, such that the PPG signal is not continuous. Thus, the PPG signal may be sampled forming a time sequence of values. A sampled PPG signal, wherein the sampling is done to represent the PPG signal in a digital form rather than for extracting features of the PPG signal, should also be construed as being a raw PPG signal, such that the DNN receives a complete dataset of a time-series of the PPG signal.

According to an embodiment, the method further comprises associating the determined subject-specific model with the specific subject.

This implies that the model is associated to the specific subject for which the model is generated. Thus, the method is configured to generate a single model for each subject, such as to form a one-to-one relationship between models and subjects.

According to an embodiment, the DNN comprises a stack of neural network layers.

The stack of neural network layers may allow different functions of the DNN to be provided in different neural network layers. The stack may then in combination enable forming of a robust model.

According to an embodiment, the stack of neural network layers comprises one or more convolutional neural network (CNN) layers, which perform automatic feature extraction of the complete dataset of the time-series of the PPG signal.

The at least one CNN layer is suitable for automatic feature extraction in the PPG signal. Thus, the relevant features for enabling estimation of heart rate may be determined by the CNN layer(s) without a prior feature extraction of the PPG signal.

Stacking of multiple CNN layers may improve feature extraction, where downstream layers may be able to capture more complex or differentiating features. Thus, the network may integrate information from different layers and various levels of abstraction.

According to an embodiment, the stack of neural network layers further comprises one or more long short term memory (LSTM) layers, which capture temporal properties of the PPG signal.

LSTM layers may be particularly useful for capturing temporal properties. Thus, the use of one or more LSTM layers in the stack of neural network layers may be advantageous in capturing temporal-feature information in the PPG signal.

The LSTM layer(s) may be suitable to use in combination with CNN layer(s). The generated features from CNN layer(s) may not be phase invariant. Depending on a time of occurrence of a heart beat within a time period of the PPG signal relative to the beginning of the time period, the relevant features may be slightly shifted. The LSTM layer(s) may thus be used in the stack in order to capture the temporal dependency of the PPG signal amidst a sequence of historical local trends of the underlying heart activity in the PPG signal and the LSTM layer(s) may thus help the stack of neural network layers to not be affected by a phase offset of features from the CNN layer(s).

According to an embodiment, the stack may comprise 2 CNN layers followed by 2 LSTM layers.

It has been found that a stack using 2 CNN layers and 2 LSTM layers may perform very well in generating a reliable model for heart rate estimation based on a PPG signal.

Selection of filter parameters in CNN and LSTM and/or selection of number of layers in the neural network may be based on a trade-off between complexity of the neural network and performance in generating a reliable model. Adding layers may imply that a higher performance is achieved, but added layers also imply that computational complexity is increased. Using a stack of 2 CNN layers followed by 2 LSTM layers enables the DNN to provide a reliable performance across different PPG data in ambulatory environments to generate a reliable model, while the computational complexity is limited.

According to an embodiment, the two CNN layers may each be formed by a one-dimensional CNN operation with Scaled Exponential Linear Unit (SELU) activation, interleaved with a pooling layer and a dropout layer. This may ensure generalization of the model, preventing the model from overfitting to the data used in generation of the model.

It should be realized that the stack of neural network layers may be implemented in different manners. For instance, in an embodiment, the stack of neural network layers may comprise 3 CNN layers followed by 1 LSTM layer. Such a stack may also be useful in generating a reliable model.

According to an embodiment, the received subject-specific training data comprises a set of PPG signals including PPG signals acquired in relation to different activities of the subject.

Thanks to the subject-specific training data being acquired in relation to different activities, the training data may include different types of artifacts and various different levels of the heart rate of the subject. This may ensure that a model may be generated which may provide a more robust heart rate estimation for various different levels of the heart rate.

According to a second aspect, there is provided a computer program product comprising a computer-readable medium with computer-readable instructions such that when executed on a processing unit the computer-readable instructions will cause the processing unit to perform the method according to the first aspect.

Effects and features of this second aspect are largely analogous to those described above in connection with the first aspect. Embodiments mentioned in relation to the first aspect are largely compatible with the second aspect.

The computer program product may implement the method in a processing unit, which may be a dedicated processing unit for performing the method or may be a general-purpose processing unit which may be able to perform the method based on the computer program product.

According to a third aspect, there is provided a method for heart rate estimation from a photoplethysmography, PPG, signal of a subject, said method comprising: acquiring subject-specific training data for machine learning, said training data comprising a PPG signal acquired by a wearable device from the subject and a heart rate indicating signal from the subject, wherein the heart rate indicating signal provides a ground truth of heart rates of the subject for associating a heart rate with a time period of the PPG signal; transferring the subject-specific training data to a machine learning process; receiving a subject-specific machine-learned model from the machine learning process, wherein the model defines a relation of a complete dataset of a time-series of a PPG signal over a time period to a heart rate of the subject; acquiring a PPG signal by the wearable device for heart rate estimation from the subject; and determining a heart rate of the subject based on the acquired PPG signal for heart rate estimation and the subject-specific model.

Effects and features of this third aspect are largely analogous to those described above in connection with the first and second aspects. Embodiments mentioned in relation to the first and second aspects are largely compatible with the third aspect.

According to a third aspect, a training phase may be used for acquiring subject-specific training data, which is used to generate a model. Then, in a deployment phase, the model may be used so as to enable heart rate estimation based on a PPG signal. Thus, a model for heart rate estimation from a PPG signal may be determined, without any need to identify and eliminate motion artifacts in the PPG signal. Rather, the model is able to correctly estimate the heart rate based on the model being formed on a complete dataset of a time-series of the PPG signal over a time period.

The method according to the third aspect may be performed in a wearable device carrying a PPG sensor. The wearable device may further include a heart rate sensor (e.g. an ECG sensor) for acquiring a heart rate indicating signal or, alternatively, the wearable device may be connected to the heart rate sensor during the training phase. For instance, a wireless connection may be established during the training phase, such that the wearable device may collect and package both heart rate indicating signals and PPG signals as subject-specific training data. The subject-specific training data may then be transferred to a machine learning process. The machine learning process may be performed in a separate, external unit, such that the wearable device may transfer the subject-specific training data to the external unit. For instance, the subject-specific training data may be transferred over a computer network, such as the Internet, so that the machine learning process may be implemented on an external unit "in the cloud". The wearable device may transfer the subject-specific training data to the computer network through a wired or wireless connection. However, the machine learning process may be performed in the wearable device itself for generating the model within the wearable device.

It should also be realized that the heart rate indicating signals of the subject-specific training data may be separately transferred to the machine learning process. Thus, the heart rate indicating signal and the PPG signal may be acquired by separate devices, which may each communicate acquired data separately to the machine learning process. For instance, the heart rate sensor may be an ECG sensor which, independently from a wearable device carrying the PPG sensor, communicates with an external unit for generating the model. Thus, the external unit may be configured to associate the heart rate indicating signal with the PPG signal in order to enable generating of the model. The heart rate indicating signal and the PPG signal may be time-stamped using synchronized clocks in order to enable correctly associating the heart rate indicating signal with the PPG signal.

According to an embodiment, the training data is acquired during an initial training period for generation of the machine-learned model, and after the subject-specific machine-learned model has been generated, the determining of the heart rate of the subject based on the acquired PPG signal for heart rate estimation is enabled.

Thus, training data may first be acquired during a training period. The model may be generated once a sufficient amount of training data has been acquired. Once a model has been generated, the model may be verified, e.g. against a part of the training data or against data acquired in an additional verification period. Thus, a verification may be performed to ensure that the model is able to reliably estimate heart rate. Then, when a reliable model has been generated, the model may be deployed to allow heart rate estimation based only on the PPG signal.

According to an embodiment, the method further comprises updating the subject-specific machine-learned model after the subject-specific machine-learned model has been initially generated, said updating comprising during an updating training period: acquiring subject-specific updating training data for machine learning, said updating training data comprising a PPG signal from the subject and a heart rate indicating signal from the subject, wherein the heart rate indicating signal provides a ground truth of heart rates of the subject for associating a heart rate with a time period of the PPG signal; transferring the subject-specific updating training data to a machine learning process; receiving an updated subject-specific machine-learned model from the machine learning process, wherein the updated model defines a relation of a complete dataset of a time-series of a PPG signal over a time period to a heart rate of the subject.

Physiological factors of a subject may change over time. Thus, additional training data may be needed from time to time to ensure that the model continues to allow correct heart rate estimation from the PPG signal. The updating training period may be periodically triggered to ensure that a reliable model is maintained. Alternatively, a subject may trigger an updating training period when suspecting that an updated model may be needed.

The updating training data may first be used to verify whether any update of the model is needed. Thus, if the model is able to reliably estimate the heart rate as may be confirmed against the ground truth of heart rates, a determination that no update is needed may be made. Thus, the initial verification based on the training data may ensure that processing resources are saved, as generation of the updated model is only triggered when necessary.

A PPG signal may be acquired by detecting interaction of light with blood flow. Light may be emitted towards a skin surface of the subject so that light will interact with the blood flow and then a light having interacted with the blood flow is detected. In a reflective mode, light being reflected is detected, whereas in a transmissive mode, light being transmitted through a body part including blood vessels is detected. Typically, the PPG signal may be based on green light or infrared light, which are suitable for detecting interaction of light with blood flow.

According to an embodiment, acquiring subject-specific training data for machine learning comprises acquiring a PPG signal from the subject using a PPG sensor emitting green light towards a skin surface of the subject and detecting reflected light from a skin surface of the subject.

Acquiring a PPG signal using green light in a reflective mode may be particularly useful, since green signals may produce large intensity variations to cardiac modulation and yield a better signal-to-noise ratio compared to use of infrared light. Thus, a strong signal may be received and/or power may be saved by using relatively short light pulses and/or relatively weak intensity of emitted light compared to a PPG signal based on infrared light and/or a transmissive mode of acquiring the PPG signal. Further, a reflective mode system may provide better user comfort than a transmissive mode system.

According to an embodiment, acquiring subject-specific training data for machine learning comprises acquiring a PPG signal from the subject using a PPG sensor and pre-processing the PPG signal.

Thus, the PPG signal acquired by the PPG sensor may first be pre-processed to remove information that does clearly not relate to heart activity. Thus, the signal may be generally cleaned, without removing any information of the heart activity from the PPG signal. Thus, the pre-processing does for instance not remove a time interval of the PPG signal in which a motion artifact or other noise is present. The pre-processed signal may enable a more efficient generation of the model, as a cleaner signal may be provided as input.

According to an embodiment, the pre-processing of the PPG signal includes filtering the PPG signal using a bandpass filter.

This implies that the PPG signal may be filtered to remove frequency components which are physically impossible to correspond to a heart rate. For instance, a filter, such as a $4^{th}$ order Butterworth filter, having cut-off frequencies of 0.1-18 Hz may be used. This may imply that high frequency noise components and DC drifts may be removed from the signal of interest.

According to an embodiment, pre-processing may also involve normalization of the filtered signal to zero mean and unit variance. This may be advantageous in further processing of the signal without removing any information of the heart activity from the signal.

According to an embodiment, acquiring subject-specific training data for machine learning comprises receiving an electrocardiogram, ECG, signal providing a heart rate indicating signal from the subject.

The ECG signal may provide a reliable heart rate, such that a reliable ground truth may be provided. The ECG signal may be received by a wearable device, which is configured to acquire the PPG signal. Thus, the subject-specific training data may be packaged within the wearable device. Alternatively, the ECG signal as well as the PPG signal may be separately received in an external unit, which is configured to execute the generation of the subject-specific model, or in an intermediate external unit, which may package the ECG signal and the PPG signal and forward the subject-specific training data to the external unit executing the generation of the subject-specific model.

According to a fourth aspect, there is provided a computer program product comprising a computer-readable medium with computer-readable instructions such that when executed on a processing unit the computer-readable instructions will cause the processing unit to perform the method according to the third aspect.

Effects and features of this fourth aspect are largely analogous to those described above in connection with the first, second and third aspects. Embodiments mentioned in relation to the first, second and third aspects are largely compatible with the fourth aspect.

The computer program product may be provided to a processing unit, which may be arranged in a wearable device comprising the PPG sensor. Thus, the computer program product may be executed to perform the method in a wearable device. However, it should be realized that the computer program product may alternatively be provided to a processing unit which is arranged in an intermediate unit. Thus, the intermediate unit may be configured to acquire PPG signals by receiving the PPG signals from a PPG sensor, which may be arranged in the wearable device, which is physically separate from the intermediate unit.

According to a fifth aspect, there is provided a system for heart rate estimation from a photoplethysmography, PPG, signal of a subject, said device comprising: a PPG sensor for detecting a PPG signal from the subject, a communication unit, which is configured to communicate with a neural network for machine learning, wherein the communication unit is configured to acquire from the PPG sensor subject-specific training data for machine learning, said training data comprising the PPG signal from the subject; the communication unit being further configured to transfer the subject-specific training data to the neural network, which further receives a heart rate indicating signal from the subject, wherein the heart rate indicating signal provides a ground truth of heart rates of the subject for associating a heart rate with a time period of the PPG signal, and the communication unit being further configured to receive a subject-specific machine-learned model from the neural network, wherein the model defines a relation of a complete dataset of a time-series of a PPG signal over a time period to a heart rate of the subject; and a processor, which is configured to receive the subject-specific model from the communication unit and, after receiving the subject-specific model, receive the PPG signal from the PPG sensor for heart rate estimation; the processor being further configured to determine a heart rate of the subject based on the received PPG signal from the PPG sensor and the subject-specific model; and a carrier having a shape adapted to be worn by a subject and configured to carry the PPG sensor, the communication unit and the processor.

Effects and features of this fifth aspect are largely analogous to those described above in connection with the first, second, third and fourth aspects. Embodiments mentioned in relation to the first, second, third and fourth aspects are largely compatible with the fifth aspect.

The system may be configured to acquire a PPG signal during a training period such that a subject-specific model may be generated and, once the model has been generated, the system may be able to estimate heart rates based merely on the PPG signal.

The system may be able to store a plurality of models for different subjects, such that the system may be shared by different subjects and enables heart rate estimation based on separately trained models. In this regard, the system may enable a subject to input identity information so as to activate the relevant model. Alternatively, the system may automatically identify the subject among the subjects for which models are stored in the device, e.g. based on subject-specific characteristics of the PPG signal.

According to an embodiment, the system further comprises a heart rate sensor, such that the system may acquire the heart rate indicating signal during the training period. Once the model has been generated, the heart rate sensor may be de-activated, e.g. for saving power, and the PPG signal may still be used to estimate heart rate.

According to another embodiment, the system further comprises a receiver, configured to be connected to a heart rate sensor for acquiring a heart rate indicating signal from the subject. For instance, the receiver may form a wired or wireless connection to the heart rate sensor for receiving the heart rate indicating signal. Thus, the system may collect subject-specific training data including PPG signals and heart rate indicating signals, without the device including a heart rate sensor.

The communication unit may thus be configured to acquire the training data from the PPG sensor and the receiver. The communication unit may then further transfer the subject-specific data comprising the PPG signal and the heart rate indicating signal to the neural network.

According to yet another embodiment, the communication unit of the device only transfers the acquired PPG signal from the subject to the neural network. The heart rate sensor may then be external to the system and may be configured to separately communicate the heart rate indicating signal to the neural network.

According to an embodiment, the system further comprises a carrier, which is configured to be worn on a wrist of a subject, wherein the PPG sensor, the communication unit and the processor are arranged in or on the carrier.

Thus, the system may be arranged in a single physical unit and may be implemented in a wearable carrier.

Thus, the system may be conveniently worn by the subject, so as to allow heart rate estimation in an unobtrusive manner, not affecting daily life of the subject.

It should be realized that the device may comprise a carrier which may be worn in another manner on the subject. For instance, the carrier may alternatively be configured to be worn on an earlobe or a fingertip of the subject.

According to an embodiment, the receiver, configured to be connected to a heart rate sensor for acquiring a heart rate indicating signal from the subject, may further be arranged in or on the carrier.

However, it should be realized that according to alternatives, the system may be distributed in a plurality of physical units. For instance, the PPG sensor may be arranged in or on the carrier, which is configured to be worn on a wrist of a subject, whereas the communication unit and the processor may be arranged in one or more separate physical units, which may or may not be worn by the subject, and may be configured to communicate with the PPG sensor for receiving PPG signals.

Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.

> Fig. 1 is a flow chart illustrating a method for generating a subject-specific model for heart rate estimation according to an embodiment.
> Fig. 2 is a flow chart illustrating a method for acquiring data for generating the model and for using the generated model according to an embodiment.
> Fig. 3 is a flow chart illustrating training and verifying of a machine-learned model according to an embodiment.
> Fig. 4 is a schematic view of a stack of neural network layers used in generating the model.
> Fig. 5 is a schematic view of a device for heart rate estimation from a PPG signal according to an embodiment.

Detailed description

The inventive concept enables heart rate estimation from a photoplethysmography (PPG) signal. A machine-learned model is generated for relating the PPG signal to heart rates, such that, once generated, heart rate estimation is enabled using only the PPG signal.

The machine-learned model may be generated in a supervised framework, wherein a heart rate indicating signal providing a ground truth of heart rate is included for assisting generation of the model. During a dedicated training phase, a relationship between time intervals of the PPG signal (in time windows or frames) and the heart rate determined from a corresponding time interval of the heart rate indicating signal (considered as ground truth) are learnt. Once trained, the model may predict the heart rate for a test set of PPG data. The predictions may be verified against the ground truth heart rate to verify that the model is effective in predicting heart rate from PPG signals.

It should be realized that other signals or information may be acquired in association with the PPG signal, but are not necessary in order for a heart rate estimation to be made.

The machine learning framework may be used in a personalized use-case scenario. The basic premise of this use-case rests on the fact that biological signals are influenced by various physiological factors - age, sex, height, weight/body-mass-index, etc. and most importantly the cardiac condition of a given subject. Thus, a neural network may be trained to build a model which learns the underlying subject-specific data distribution.

For better generalization, the model could learn possible variations inherent within the data. Hence, the model may be cross-validated over a training dataset and prospectively evaluated on test data.

Thanks to the generation of a subject-specific model being determined, manual extraction of features in the PPG signal may not be needed in order to enable a reliable model to be formed. Also, extensive processing of the PPG signal to reduce or remove motion artifacts or other noise may not be needed to be performed before training of the model. Thus, the input for training may include all information available in the PPG signal so as to allow the model to consider the entire PPG signal in the generation of the model. This may allow a robust and reliable model to be generated.

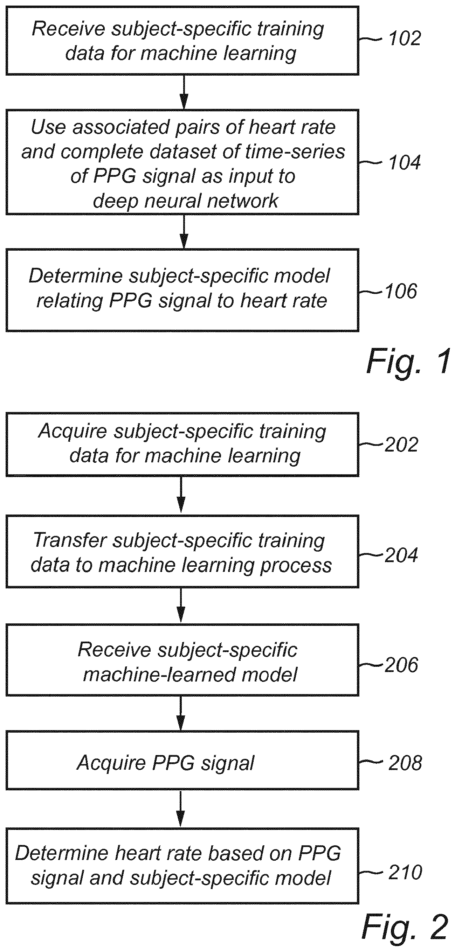

Referring now to Fig. 1, a method of generating the model for the heart rate estimation will be generally described.

The method comprises receiving, step 102, subject-specific training data for machine learning. The subject-specific training data may be received by any processing unit which may be configured to perform machine learning. The processing unit may comprise extensive computing power in order to enable performing of the machine learning. For instance, the processing unit may be implemented as any computer unit (or network of computer units) which may be connected to a communication network for receiving the training data. Thus, the processing unit may be implemented "in the cloud". The processing unit may be configured to receive the training data from sensor(s) used for acquiring the training data. The communication of the training data may take place via a number of intermediate nodes, such that the training data may e.g. be provided via the Internet.

The training data may comprise a PPG signal from the subject and a heart rate indicating signal from the subject, wherein the heart rate indicating signal provides a ground truth of heart rates of the subject for associating a heart rate with a time period of the PPG signal.

The method further comprises using, step 104, associated pairs of a heart rate and a complete dataset of a time-series of a PPG signal over the time period as input to a deep neural network (DNN). The DNN may be implemented in the processing unit described above. Thus, the DNN may receive information of heart rates in time frames that are related to the PPG signals in the corresponding time frames for training the DNN to generate a model. The PPG signal is provided as a raw signal, which may possibly have been pre-processed. The information relating to heart activity in the PPG signal is maintained, such that the complete dataset of the time-series of the PPG signal forms a signal which is continuous in time by including periodical samples in the time-series.

The method further comprises determining, through the DNN, step 106, a subject-specific model relating the PPG signal of the subject to the heart rate of the subject. The determination of the subject-specific model may thus utilize the complete datasets of the PPG signal over time frames to generate a reliable model for enabling heart rate estimation from PPG signals of the subject.

Referring now to Fig. 2, a method of using the model will be described. Since a subject-specific model is generated, training data may first need to be acquired from the subject and then the generated model may be deployed for use for the subject. This implies that the same device may be used for acquiring training data and may later receive the model for enabling heart rate estimation based on a PPG signal.

Thus, the method may comprise acquiring, step 202, subject-specific training data for machine learning. The training data comprises a PPG signal from the subject and a heart rate indicating signal from the subject, wherein the heart rate indicating signal provides a ground truth of heart rates of the subject for associating a heart rate with a time period of the PPG signal.

The acquiring of subject-specific training data may be controlled by a device which comprises a PPG sensor, such as a wearable device, e.g. a wristband. The device may acquire the PPG signal through the PPG sensor. Further, the device may acquire the heart rate indicating signal by receiving the heart rate indicating signal from a heart rate sensor, such as an ECG sensor, which may be arranged in a different physical unit. Thus, the heart sensor may be configured to communicate with the device comprising the PPG sensor for transferring detected heart rate indicating signals to the device through wired or wireless communication.

As an alternative, the acquiring of subject-specific training data may be controlled by a central unit. The central unit may acquire the PPG signal and the heart rate indicating signal by receiving the PPG signal from a PPG sensor and the heart rate indicating signal from a heart rate sensor. The PPG sensor, the heart rate sensor and the central unit may each be arranged in different physical units, and may be configured for wired or wireless communication for transferring PPG signals and heart rate indicating signals to the central unit. For instance, the central unit may be configured as a mobile phone, which may communicate with the PPG sensor and the heart rate indicating signal. The central unit may comprise a processor, which provides processing resources for performing heart rate estimation.

The method may further comprise transferring, step 204, the subject-specific training data to a machine learning process. Thus, the subject-specific training data may be transferred to a neural network, which may be configured to perform the machine learning process.

The neural network may be implemented in a computer unit, which may be remote from the PPG sensor and the heart rate sensor. The neural network may be configured to receive subject-specific training data relating to different subjects, such that the same neural network may perform machine learning for different subjects spread out across the world. Thus, the subject-specific training data may be transferred through a computer and/or telecommunication network in order to reach the neural network. However, it should also be realized that the neural network may be implemented by a subject receiving a software executing the neural network for installation on a computer unit, such as a personal computer or a mobile phone, owned by the subject. Thus, the transferring of the subject-specific training data may comprise communicating the subject-specific training data over a direct communication link between the PPG sensor and the heart rate sensor and the computer unit on which the neural network is installed. As yet another alternative, the neural network may even be installed in a device carrying the PPG sensor and/or the heart rate sensor, such that the transfer of the subject-specific training data to the neural network may comprise transfer of data between processes in the device.

The PPG signals and the heart rate indicating signals may be formed into one or more combined packages for transferring the PPG signals and the heart rate indicating signals together to the machine learning process. However, as an alternative, the PPG signals and the heart rate indicating signals may be separately transferred, such that the transfer of the subject-specific data comprises transferring the PPG signal and transferring the heart rate indicating signal. The PPG signal may be transferred by a communication unit associated with the PPG sensor, whereas the heart rate indicating signal may be transferred by a communication unit associated with a heart rate indicating signal. Thus, the PPG signals and the heart rate indicating signals may be separately received by a neural network, which may associate the signals therein for performing the machine learning process.

The method further comprises receiving, step 206, a subject-specific machine-learned model from the machine learning process. Thus, the model may be provided to a processor in which heart rate estimation is to be performed based on the PPG signal. For instance, the processor may be arranged in a device carrying the PPG signal or in another device which is worn by the subject or in vicinity of the subject, such that the heart rate may be estimated and displayed to the subject.

Thus, the method may further comprise acquiring, step 208, a PPG signal for heart rate estimation from the subject; and determining, step 210, a heart rate of the subject based on the acquired PPG signal for heart rate estimation and the subject-specific model.

Using the model, the heart rate may be estimated based only on the PPG signal. Thus, once the model is deployed, heart rate estimation may be performed while the subject only wears a PPG sensor.

The acquiring of the PPG signal and determining of the heart rate may be performed by the device comprising the PPG sensor. The device may thus also comprise the processor that receives the model. However, as an alternative, the model may be deployed on a processor which may be arranged in a separate physical unit from the PPG sensor, such as a mobile phone or a computer unit of the subject. Thus, acquiring of the PPG signal may comprise receiving the PPG signal from the PPG sensor by the processor, which then determines the heart rate of the subject.

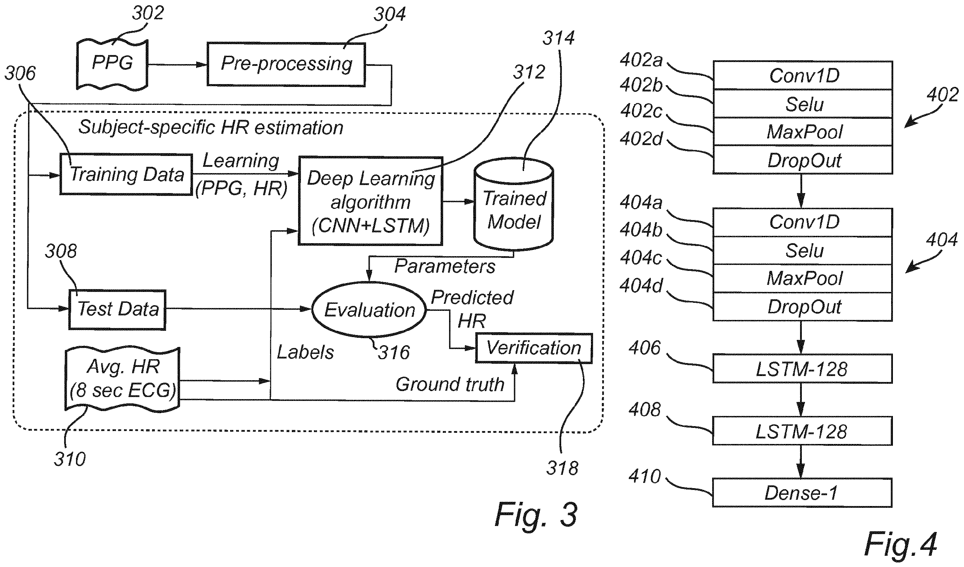

Referring now to Fig. 3, as a complement to the above description, an overview of training and verifying a machine-learned model is described.

A PPG signal is acquired, step 302. The PPG signal may be pre-processed, step 304. The PPG data samples may be pre-processed with a band-pass 4th order Butterworth filter having the cut-off frequencies 0.1 - 18 Hz, which primarily restricts the high frequency noise component and DC drifts from the signal of interest. The filtered signal may further be normalized to zero mean and unit variance.

The PPG signal may thus comprise a signal which is continuous in time (samples are equally spaced). The PPG signal may be divided into time periods, which may also be called windows or frames. Each window may be 8 seconds long, but it should be realized that other lengths of the periods may be used. The windows may be arranged in sequence or may be partially overlapping. For instance, a 2 second sliding window may be used, i.e. there may be a 6 seconds overlap between two sequential windows being 8 seconds long. The length of the window may advantageously be sufficiently long to ensure that a heart rate may be accurately determined within the time period, while being sufficiently short so that the heart rate does not vary within the time period.

The PPG signal may be formed into training data, step 306, and test data, step 308. The training data may be used for training the neural network, whereas the test data may be used for testing the model to verify whether the model is successful in predicting the heart rate from the PPG signal.

Further, a heart rate indicating signal may be acquired, step 310, to provide a ground truth of the heart rate. The heart rate indicating signal may for instance be acquired as an ECG signal. Further, the heart rate indicating signal may also be divided into windows in the same manner as for the PPG signal such that a ground truth value of the heart rate may be provided for each PPG signal window.

Then, the training data part of the PPG signal and the heart rate indicating signal may be transferred to a neural network for forming the machine-learned model. The training data may associate PPG signal windows with heart rate information for corresponding windows of the heart rate indicating signal. The heart rate information may thus provide a label of the heart rate for each PPG signal window. The neural network may apply a deep learning algorithm, step 312, on the training data to determine relations between PPG signal and heart rate.

Thus, a trained model is generated, step 314. The trained model may provide parameters which are to be used for estimating heart rate from PPG signals.

Then, the test data and the parameters of the model are provided for evaluation, step 316, of the machine-learned model. In the evaluation, heart rates are predicted using the machine-learned model on the PPG signals of the test data.

The predicted heart rate values are used for verification, step 318, of the machine-learned model. In verification, ground truth values corresponding to the test data are provided from the heart rate indicating signal, so that a comparison may be made whether the predicted values correspond to the actual heart rate values. Once the model is able to satisfactorily predict heart rate values as determined in the evaluation and verification, the machine-learned model may be deployed. Then, new PPG signals may be acquired, without any need of acquiring other information, and heart rate may be estimated based on the new PPG signals and the deployed machine-learned model.

The machine-learned model may be periodically updated in order to ensure that the machine-learned model continues to reliably predict heart rates from PPG signals of the subject. The updating of the machine-learned model may for instance adapt the machine-learned model to changing physiological factors of the subject.

The updating may e.g. be performed on a yearly basis.

The updating of the machine-learned model may comprise acquiring PPG signals and heart rate indicating signals providing ground truth. The PPG signals may first be used as test data to evaluate, step 316, the existing machine-learned model based on the acquired PPG signals. The predicted heart rates may be verified, step 318, against the ground truth provided by the heart rate indicating signals to determine whether the model reliably predicts heart rates. If not, a new training period may be commenced, wherein PPG signals are acquired to form training data, steps 302, 304, 306, and heart rate indicating signals are acquired to provide ground truth, step 310. Then, the new training data may be transferred to the DNN for generating the machine learned model, steps 312, 314. The generation of the machine-learned model may start in the existing model to improve the model or may be generated from scratch using only the new training data.

Referring now to Fig. 4, an embodiment of the deep neural network (DNN) used for generating the model will be described in further detail.

A DNN may be configured to provide machine learning, wherein the neural network automatically discovers representations needed for feature detection or classification from raw data, so that manual feature engineering may not be needed and the neural network is able to both learn features and then use the features to perform a specific task.

A DNN may include multi-layer perceptrons (MLP), convolutional neural networks (CNN), and/or Recurrent Neural Networks (RNN). In the present embodiment, CNN layers and long-short term memory (LSTM) layers, which are a variant of RNN, are used for generating the model. It has been found that such a stack of layers in the DNN is particularly efficient in forming a reliable model for heart rate estimation from PPG signals. However, it should be realized that other combination of layers may be used to also form a satisfactory model.

CNNs may comprise an initial layer of convolutional filters (a set of weights which slides over the input), followed by non-linearity (activation function - rectified linear units), sub-sampling (pooling), and a fully connected layer which realizes a classification. The stacking of multiple convolutional layers helps to achieve automatic feature extraction, where downstream layers capture more complex or differentiating features. This helps the network to integrate information from different filters and various levels of abstraction.

RNNs are an effective choice for analyzing time series data for inferring sequential/time-variant information, since they incorporate contextual information from past inputs and are robust to localized distortions in the input sequence along time.

LSTM, uses a memory block inspired by a computer memory cell, where context-dependent input, output, and forget gates control what is written to, read from, and kept in the cell in each time-step. Hence, it becomes convenient for the network to store a given input over many time steps, in effect helping LSTM layers to capture temporal properties.

In the embodiment illustrated in Fig. 4, a data driven approach is realized by allowing the network to learn discriminatory features of the PPG signal, accomplished by using a first and a second layer 402, 404 of a one-dimensional CNN (1-D CNN).

The 1-D CNN layers 402, 404 can be thought of as a feature extractor. The input is convolved with the filters to generate points in the temporal-feature domain. Corresponding layers use these features to convolve with additional filters to generate the final features from the time-series PPG input.

A potential drawback of CNN is that the generated features are not completely phase invariant. Depending on the time of occurrence of the heart beat relative to the beginning of the sample, the relevant features will be slightly shifted. Hence, LSTM being instrumental in capturing the temporal dependency amidst the sequence of historical local trends of the underlying cardiac activity in PPG signals, further helps to recover from the phase offset.

Thus, the output from the second 1-D CNN layer 404 is then fed into a first and a second LSTM layer 406, 408. Finally, output from the second LSTM layer 408 is passed through a dense layer 410 which is customized for the application. Dense layers consist of a set of weights for each neuron that is multiplied by the input and summed to give the neuron's activation. The dense layer 410 may use a single neuron with linear transfer function (activation) on the LSTM output to estimate a continuous HR value.

Each CNN layer 402, 404 may comprise a 1-D CNN operation 402a, 404a with Rectified Linear Unit (Re-LU) activation 402b, 404b, e.g. a Scaled Exponential Linear Unit (SELU) activation.

Further, each CNN layer 402, 404 may comprise a batch normalization layer preceding the RELU activation.

Also, each CNN layer 402, 404 may comprise a max-pooling layer 402c, 404c. The max-pooling layer may for instance use a pool size of 4.

Each CNN layer 402, 404 may further comprise a dropout layer 402d, 404d for reducing overfitting. The dropout layer may for instance use a dropout rate of 0.1.

Each LSTM layer 406, 408 may use hyperbolic tangent (tanh) function.

Also, each LSTM layer 406, 408 may use a Root Mean Square Propagation (RMSProp) as an optimizer with default hyperparameters.

The topology of the DNN in Fig. 4 was implemented using a filter size of 40 ($sF$) in both CNN layers 402, 404, with number of filters as 32 ($nF$). A size of 128 units ($nU$) were selected for both LSTM layers 406, 408 working in conjunction with the CNN layers 402, 404. These hyperparameters were optimized using a heuristic grid search method, and selected parameters yielding the best performance. Increasing $sF$, $nF$, $nU$ or the number of layers ($nL$) beyond the ones selected here did not result in an improved overall performance.

The DNN algorithm discussed above was evaluated on the IEEE Signal Processing Cup (SPC) 2015 database, see Zhilin Zhang, Zhouyue Pi, Benyuan Liu, "TROIKA: A General Framework for Heart Rate Monitoring Using Wrist-Type Photoplethysmographic Signals During Intensive Physical Exercise", IEEE Transaction on Biomedical Engineering, vol. 62, no. 2, pages 522-531, February 2015. The IEEE SPC 2015 database comprises PPG signals of 5-minute duration, from 20 healthy subjects, age ranging 18 to 58.

Each subject's data contains two channels of PPG, recorded from the wrist (dorsal) using a pulse oximeter with green LED (wavelength: 515 nm); tri-axial accelerometer signals also recorded from the wrist, and a channel of ECG recorded from the chest using wet ECG electrodes, all recorded simultaneously. The ECG signal acts as the ground-truth for PPG-based heart rate estimation.

All signals were sampled at 125 Hz. PPG window (frame) lengths considered for this evaluation of the DNN was 8s (sliding by 2s), like ECG-HR computation. The subjects performed three types of activities.

First, Type1 (T1), performed by subjects 1-12, involving walking or running on a treadmill with the following speeds in order: 1-2 km/h for 0.5 min, 6-8 km/h for 1 min, 12-15 km/h for 1 min, 6-8 km/h for 1 min, 12-15 km/h for 1 min, and 1-2 km/h for 0.5 min. The subjects used their hand (with wristband) to pull clothes, wipe sweat on forehead, and push buttons on the treadmill.

Second, Type2 (T2), performed by subjects 13, 14, 15, 18 and 20, involved in forearm/upper arm exercises (e.g. shake hands, stretch, push, running, jump, and push-ups).

Last, Type3 (T3), performed by subjects 13, 15, 16, 17, 18 and 19, involving intense arm movements (e.g. boxing). Hence, there are 20 subjects and 23 records in total, since subjects 13, 15 and 18 were involved in both T2 and T3.

The performance of the DNN described above was evaluated with respect to the data of the IEEE SPC 2015 database.

A metric considered for heart rate estimation is absolute error ($AE$), computed as an absolute difference between the true heart rate from ECG ($HR_E$) and the estimated heart rate from PPG ($HR_P$), which was computed as:

$$AE_i = \mathrm{abs}(HR_E - HR_P)$$

where i denotes a window within the acquired signals.

Correspondingly, the mean absolute error ($MAE$) and the standard deviation of the absolute error ($SDAE$) are computed. Here, the metrics have been computed for the first 12 subjects performing T1, records 14-23 involved in T2 and T3, and finally the results for all 23 records. A $MAE \pm SDAE$ of $0.52 \pm 0.19$ beats per minute (BPM) was achieved for records 1-12, a $MAE \pm SDAE$ of $0.44 \pm 0.18$ BPM was achieved for records 14-23 and a $MAE \pm SDAE$ of $0.48 \pm 0.19$ was achieved for records 1-23. These results are an improvement over other reported results for the data of the IEEE SPC 2015 database.

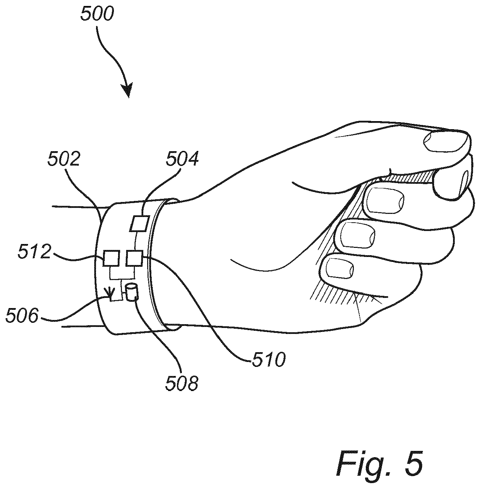

Referring now to Fig. 5, a device 500 will be described. The device 500 may be used for acquiring at least the PPG signal for subject-specific training data and may receive a machine-learned model based on the training data such that the device 500 may, after a training period, be used for estimating heart rates based on acquired PPG signals.

The device 500 may comprise a carrier 502, which may have a shape adapted for being worn by a subject. For instance, the carrier 502 may be in the form of a wristband, which may be worn on a wrist of the subject.

The carrier 502 may be configured to carry components of the device 500 such that the components are provided to be worn by the subject.

The device 500 may further comprise a PPG sensor 504. The PPG sensor 504 may comprise a light source for emitting light and a light sensor for detecting an intensity of light. The PPG sensor 504 may be mounted in or on the carrier 502 so as to enable the light source to emit light towards a skin surface of the subject so that light will interact with blood flow in the subject. The light sensor is arranged to detect light having interacted with the blood flow. The detected light will thus be modulated by the heart pulse of the subject, such that the heart rate may be estimated using the machine-learned model.

The PPG sensor 504 may be arranged so that reflected light is detected by the light sensor. However, it should be realized that the PPG sensor 504 may alternatively be arranged in the carrier 502 so that transmitted light is detected, e.g. by arranging the light source and the light sensor on opposite sides of a body part of the subject. Using a transmissive mode may be particularly feasible if the carrier 502 is to be worn around a relatively thin body part, such as a fingertip or an earlobe.

The light source of the PPG sensor 504 may be configured to emit green light, which may produce large intensity variations based on modulation by the heart pulse of the subject. However, it should be realized that other wavelengths of light may be used, such as infrared light.

The device 500 may further comprise a communication unit 506, which may be configured to communicate with a neural network for transferring subject-specific training data to the neural network. The communication unit 506 may be configured for wired or wireless communication. For instance, the communication unit 506 may be configured to transfer training data to a specific external unit to which a connection is set up. Then, the specific external unit may forward the training data to the neural network, e.g. over a computer and/or telecommunication network. The communication unit 506 may for instance be paired with the specific external unit for wireless communication therewith or the device 500 may be docked with the specific external unit for providing training data via a wired connection.

The communication unit 506 may further be configured to receive the machine-learned model back from the neural network to deploy the machine-learned model in the device 500.

The device 500 may thus store the machine-learned model in a memory 508.

The device 500 may further comprise a processor 510, which is configured to access the machine-learned model. The processor 510 may further be configured to receive PPG signals from the PPG sensor 504. The processor may then process the PPG signals based on the machine-learned model and may determine an estimated heart rate.

The device 500 may further comprise a display and the processor 510 may be configured to output estimated heart rates to the display so as to allow the subject to be informed of current heart rates.

The device 500 may further comprise a receiver 512, which may be configured to be connected to a heart rate sensor for acquiring a heart rate indicating signal from the subject, which may provide a ground truth for subject-specific training.

The heart rate sensor may be part of the device 500 and may be arranged in or on the carrier 502. However, the heart rate sensor may be arranged in a separate physical unit and need not be part of the device 500.

The receiver 512 may thus receive the heart rate indicating signal via the communication unit 506, which may be configured to communicate with the heart rate sensor, e.g. over a wireless communication. The receiver 512 may be implemented as a process within the device 500, e.g. executed by the processor 508, wherein the heart rate indicating signal data may e.g. be registered into the memory 508 for enabling the data to be combined or associated with PPG signals.

The device 500 may further be configured to combine PPG signals and heart rate indicating signals into common package(s) for transferring the subject-specific training data to the neural network.

According to an alternative, the device 500 need not comprise the receiver 512. Rather, the heart rate sensor may be configured to separately transfer heart rate indicating signals to the neural network, which may then associate the heart rate indicating signals with the PPG signals.

The processor 510 may be implemented as a processing unit, such as a central processing unit (CPU), which may execute the instructions of one or more computer programs in order to implement functionality of the device 500.

The processor 510 may alternatively be implemented as firmware arranged e.g. in an embedded system, or as a specifically designed processing unit, such as an Application-Specific Integrated Circuit (ASIC) or a Field-Programmable Gate Array (FPGA), which may be configured to implement functionality of the device 500.

In the above the inventive concept has mainly been described with reference to a limited number of examples. However, as is readily appreciated by a person skilled in the art, other examples than the ones disclosed above are equally possible within the scope of the inventive concept, as defined by the appended claims.

For instance, the DNN may be implemented using other layers in the stack of neural network layers.

According to an alternative, the DNN may comprise 3 CNN layers followed by 1 LSTM layer.

**Claims**

1. A computer-implemented method of generating a model for heart rate estimation from a photoplethysmography, PPG, signal of a subject, said method comprising:

   receiving (102) subject-specific training data for machine learning, said training data comprising a PPG signal acquired by a wearable device from the subject and a heart rate indicating signal from the subject, wherein the heart rate indicating signal provides a ground truth of heart rates of the subject for associating a heart rate with a time period of the PPG signal;
   using (104) associated pairs of a heart rate and a complete dataset of a time-series of a PPG signal over a time period as input to a deep neural network, DNN; and
   determining (106; 312), through the DNN, a subject-specific model relating the PPG signal of the subject to the heart rate of the subject.

2. The computer-implemented method according to claim 1, further comprising associating the determined subject-specific model with the specific subject.

3. The computer-implemented method according to any one of claims 1 or 2, wherein the DNN comprises a stack of neural network layers.

4. The computer-implemented method according to claim 3, wherein the stack of neural network layers comprises one or more convolutional neural network, CNN, layers (402; 404), which perform automatic feature extraction of the complete dataset of the time-series of the PPG signal.

5. The computer-implemented method according to claim 4, wherein the stack of neural network layers further comprises one or more long short term memory, LSTM, layers (406; 408), which capture temporal properties of the PPG signal.

6. The computer-implemented method according to any one of the preceding claims, wherein the received subject-specific training data comprises a set of PPG signals including PPG signals acquired in relation to different activities of the subject.

7. A method for heart rate estimation from a photoplethysmography, PPG, signal of a subject, said method comprising:

   acquiring (202; 302,304, 306, 310) subject-specific training data for machine learning, said training data comprising a PPG signal acquired by a wearable device from the subject and a heart rate indicating signal from the subject, wherein the heart rate indicating signal provides a ground truth of heart rates of the subject for associating a heart rate with a time period of the PPG signal;
   transferring (204) the subject-specific training data to a machine learning process;
   receiving (206) a subject-specific machine-learned model from the machine learning process, wherein the model defines a relation of a complete dataset of a time-series of a PPG signal over a time period to a heart rate of the subject;
   acquiring (208) a PPG signal by the wearable device for heart rate estimation from the subject; and
   determining (210) a heart rate of the subject based on the acquired PPG signal for heart rate estimation and the subject-specific model.

8. The method according to claim 7, wherein the training data is acquired during an initial training period for generation of the machine-learned model, and after the subject-specific machine-learned model has been generated, the determining of the heart rate of the subject based on the acquired PPG signal for heart rate estimation is enabled.

9. The method according to claim 8, further comprising updating the subject-specific machine-learned model after the subject-specific machine-learned model has been initially generated, said updating comprising during an updating training period:

   acquiring subject-specific updating training data for machine learning, said updating training data comprising a PPG signal from the subject and a heart rate indicating signal from the subject, wherein the heart rate indicating signal provides a ground truth of heart rates of the subject for associating a heart rate with a time period of the PPG signal;
   transferring the subject-specific updating training data to a machine learning process;
   receiving an updated subject-specific machine-learned model from the machine learning process, wherein the updated model defines a relation of a complete dataset of a time-series of a PPG signal over a time period to a heart rate of the subject.

10. The method according to any one of claims 7-9, wherein acquiring (202; 302, 304, 306, 310) subject-specific training data for machine learning comprises acquiring (302) a PPG signal from the subject using a PPG sensor emitting green light towards a skin surface of the subject and detecting reflected light from a skin surface of the subject.

11. The method according to any one of claims 7-10, wherein acquiring (202; 302, 304, 306, 310) subject-specific training data for machine learning comprises acquiring (302) a PPG signal from the subject using a PPG sensor and pre-processing (304) the PPG signal.

12. The method according to claim 11, wherein the pre-processing (304) of the PPG signal includes filtering the PPG signal using a bandpass filter.

13. The method according to any one of claims 7-10, wherein acquiring (202; 302, 304, 306, 310) subject-specific training data for machine learning comprises receiving (310) an electrocardiogram, ECG, signal providing the heart rate indicating signal from the subject.

14. A system (500) for heart rate estimation from a photoplethysmography, PPG, signal of a subject, said system (500) comprising:

a PPG sensor (504) for detecting a PPG signal from the subject,
a communication unit (506), which is configured to communicate with a neural network for machine learning, wherein the communication unit (506) is configured to acquire from the PPG sensor (504) subject-specific training data for machine learning, said training data comprising the PPG signal from the subject; the communication unit (506) being further configured to transfer the subject-specific training data to the neural network, which further receives a heart rate indicating signal from the subject, wherein the heart rate indicating signal provides a ground truth of heart rates of the subject for associating a heart rate with a time period of the PPG signal, and the communication unit (506) being further configured to receive a subject-specific machine-learned model from the neural network, wherein the model defines a relation of a complete dataset of a time-series of a PPG signal over a time period to a heart rate of the subject; and
a processor (510), which is configured to receive the subject-specific model from the communication unit (506) and, after receiving the subject-specific model, receive the PPG signal from the PPG sensor (504) for heart rate estimation; the processor (510) being further configured to determine a heart rate of the subject based on the received PPG signal from the PPG sensor (504) and the subject-specific model; and
a carrier having a shape adapted to be worn by a subject and configured to carry the PPG sensor (504), the communication unit (506), and the processor (510).

15. The system according to claim 14, wherein the carrier (502) is configured to be worn on a wrist of a subject.

**Patentansprüche**

1. Computerumgesetztes Verfahren zum Generieren eines Modells zur Herzfrequenzschätzung aus einem Photoplethysmographie-, PPG, Signal einer Person, wobei das Verfahren umfasst:

Empfangen (102) von personenspezifischen Trainingsdaten zum maschinellen Lernen, wobei die Trainingsdaten ein PPG-Signal, das durch eine anziehbare Vorrichtung von der Person erfasst wird, und ein die Herzfrequenz angebendes Signal von der Person umfassen, wobei das die Herzfrequenz angebende Signal grundliegende Referenzdaten von Herzfrequenzen der Person bereitstellt, um eine Herzfrequenz einem Zeitraum des PPG-Signals zuzuordnen;
Verwenden (104) zugeordneter Paare aus einer Herzfrequenz und einem vollständigen Datensatz einer Zeitreihe eines PPG-Signals im Verlauf eines Zeitraums als Eingabe in ein tiefes neuronales Netzwerk, DNN; und
Bestimmen (106; 312), über das DNN, eines personenspezifischen Modells, welches das PPG-Signal der Person mit der Herzfrequenz der Person in Zusammenhang bringt.

2. Computerumgesetztes Verfahren nach Anspruch 1, ferner umfassend das Zuordnen des bestimmten personenspezifischen Modells zu einer spezifischen Person.

3. Computerumgesetztes Verfahren nach einem der Ansprüche 1 oder 2, wobei das DNN einen Stapel von neuronalen Netzwerkschichten umfasst.

4. Computerumgesetztes Verfahren nach Anspruch 3, wobei der Stapel von neuronalen Netzwerkschichten eine oder mehrere neuronale Faltungsnetzwerk-, CNN, Schichten (402; 404) umfasst, die eine automatische Merkmalsextraktion des vollständigen Datensatzes der Zeitreihe des PPG-Signals ausführen.

5. Computerumgesetztes Verfahren nach Anspruch 4, wobei der Stapel von neuronalen Netzwerkschichten ferner eine oder mehrere langfristige Speicher-, LSTM, Schichten (406; 408) umfasst, welche die zeitlichen Eigenschaften des PPG-Signals aufgreifen.

6. Computerumgesetztes Verfahren nach einem der vorhergehenden Ansprüche, wobei die empfangenen personenspezifischen Trainingsdaten einen Satz von PPG-Signalen umfassen, die PPG-Signale umfassen, die bezüglich unterschiedlicher Tätigkeiten der Person erfasst werden.

7. Verfahren zur Herzfrequenzschätzung aus einem Photoplethysmographie-, PPG, Signal einer Person, wobei das Verfahren umfasst:

Erfassen (202; 302, 304, 306, 310) von personenspezifischen Trainingsdaten zum maschinellen Lernen, wobei die Trainingsdaten ein PPG-Signal, das durch eine anziehbare Vorrichtung von der Person erfasst wird, und ein die Herzfrequenz angebendes Signal von der Person umfassen, wobei das die Herzfrequenz angebende Signal grundliegende Referenzdaten von Herzfrequenzen der Person bereitstellt, um eine Herzfrequenz einem Zeitraum des PPG-Signals zuzuordnen;

Übertragen (204) der personenspezifischen Trainingsdaten an einen maschinellen Lernprozess;

Empfangen (206) eines personenspezifischen maschinell eingelernten Modells von dem maschinellen Lernprozess, wobei das Modell eine Beziehung eines vollständigen Datensatzes einer Zeitreihe eines PPG-Signals im Verlauf eines Zeitraums zu einer Herzfrequenz der Person definiert;

Erfassen (208) eines PPG-Signals durch die anziehbare Vorrichtung zur Herzfrequenzschätzung der Person; und

Bestimmen (210) einer Herzfrequenz der Person basierend auf dem erfassten PPG-Signal zur Herzfrequenzschätzung und dem personenspezifischen Modell.

8. Verfahren nach Anspruch 7, wobei die Trainingsdaten während einer anfänglichen Trainingsperiode zur Generierung des maschinell eingelernten Modells erfasst werden, und nachdem das personenspezifische maschinelle Lernmodell generiert wurde, das Bestimmen der Herzfrequenz der Person basierend auf dem erfassten PPG-Signal zur Herzfrequenzschätzung aktiviert wird.

9. Verfahren nach Anspruch 8, ferner umfassend das Aktualisieren des personenspezifischen maschinell eingelernten Modells, nachdem das personenspezifische maschinell eingelernte Modell anfänglich generiert wurde, wobei das Aktualisieren während einer Aktualisierungstrainingsperiode umfasst:

Erfassen von personenspezifischen Aktualisierungstrainingsdaten zum maschinellen Lernen, wobei die Aktualisierungstrainingsdaten ein PPG-Signal von der Person und ein die Herzfrequenz angebendes Signal von der Person umfassen, wobei das die Herzfrequenz angebende Signal grundliegende Referenzdaten von Herzfrequenzen der Person bereitstellt, um eine Herzfrequenz einem Zeitraum des PPG-Signals zuzuordnen;

Übertragen der personenspezifischen Aktualisierungstrainingsdaten an einen maschinellen Lernprozess;

Empfangen eines aktualisierten personenspezifischen maschinell eingelernten Modells von dem maschinellen Lernprozess, wobei das aktualisierte Modell eine Beziehung eines vollständigen Datensatzes einer Zeitreihe eines PPG-Signals im Verlauf eines Zeitraums zu einer Herzfrequenz der Person definiert.

10. Verfahren nach einem der Ansprüche 7 bis 9, wobei das Erfassen (202; 302, 304, 306, 310) von personenspezifischen Trainingsdaten zum maschinellen Lernen das Erfassen (302) eines PPG-Signals von der Person unter Verwendung eines PPG-Sensors umfasst, der grünes Licht in Richtung auf eine Hautoberfläche der Person emittiert und reflektiertes Licht von einer Hautoberfläche der Person detektiert.

11. Verfahren nach einem der Ansprüche 7 bis 10, wobei das Erfassen (202; 302, 304, 306, 310) von personenspezifischen Trainingsdaten zum maschinellen Lernen das Erfassen (302) eines PPG-Signals von der Person unter Verwendung eines PPG-Sensors und das Vorverarbeiten (304) des PPG-Signals umfasst.

12. Verfahren nach Anspruch 11, wobei das Vorverarbeiten (304) des PPG-Signals das Filtern des PPG-Signals unter Verwendung eines Bandpassfilters umfasst.

13. Verfahren nach einem der Ansprüche 7 bis 10, wobei das Erfassen (202; 302, 304, 306, 310) von personenspezifischen Trainingsdaten zum maschinellen Lernen das Empfangen (310) eines Elektrokardiogramm-, EKG, Signals, welches das die Herzfrequenz angebende Signal der Person bereitstellt, umfasst.

14. System (500) zur Herzfrequenzschätzung aus einem Photoplethysmographie-, PPG, Signal einer Person, wobei das System (500) umfasst:

einen PPG-Sensor (504) zum Detektieren eines PPG-Signals von der Person,

eine Kommunikationseinheit (506), die dazu konfiguriert ist, mit einem neuronalen Netzwerk zum maschinellen Lernen zu kommunizieren, wobei die Kommunikationseinheit (506) dazu konfiguriert ist, von dem PPG-Sensor (504) personenspezifische Trainingsdaten zum maschinellen Lernen zu erfassen, wobei die Trainingsdaten das PPG-Signal von der Person umfassen; wobei die Kommunikationseinheit (506) ferner dazu konfiguriert ist, die personenspezifischen Trainingsdaten an das neuronale Netzwerk zu übertragen, das ferner ein die Herzfrequenz angebendes Signal von der Person empfängt, wobei das die Herzfrequenz angebende Signal grundlegende Referenzdaten der Herzfrequenzen der Person bereitstellt, um eine Herzfrequenz einem Zeitraum des PPG-Signals zuzuordnen, und die Kommunikationseinheit (506) ferner dazu konfiguriert ist, ein personenspezifisches maschinell eingelerntes Modell aus dem neuronalen Netzwerk zu empfangen, wobei das Modell eine Beziehung eines vollständigen Datensatzes einer Zeitreihe eines PPG-Signals im Verlauf eines Zeitraums zu einer Herzfrequenz der Person definiert; und

einen Prozessor (510), der dazu konfiguriert ist, das personenspezifische Modell von der Kommunikationseinheit (506) zu empfangen, und nach dem Empfangen des personenspezifischen Modells das PPG-Signal von dem PPG-Sensor (504) zur Herzfrequenzschätzung zu empfangen; wobei der Prozessor (510) ferner dazu konfiguriert ist, eine Herzfrequenz der Person basierend auf dem empfangenen PPG-Signal von dem PPG-Sensor (504) und dem personenspezifischen Modell zu bestimmen; und einen Träger, der eine Form aufweist, die dazu geeignet ist, von einer Person getragen zu werden, und dazu konfiguriert ist, den PPG-Sensor (504), die Kommunikationseinheit (506) und den Prozessor (510) zu tragen.

15. System nach Anspruch 14, wobei der Träger (502) dazu konfiguriert ist, an einem Handgelenk einer Person getragen zu werden.

**Revendications**

1. Procédé mis en oeuvre par ordinateur pour générer un modèle pour l'estimation de la fréquence cardiaque à partir d'un signal de photopléthysmographie, PPG, d'un sujet, ledit procédé comprenant de :

recevoir (102) des données d'apprentissage spécifiques à un sujet pour l'apprentissage automatique, lesdites données d'apprentissage comprenant un signal PPG acquis par un dispositif portable provenant du sujet et un signal indiquant la fréquence cardiaque provenant du sujet, le signal indiquant la fréquence cardiaque fournissant une vérité de terrain des fréquences cardiaques du sujet pour associer une fréquence cardiaque à une période de temps du signal PPG ;

utiliser (104) des paires associées d'une fréquence cardiaque et d'un ensemble de données complet d'une série temporelle d'un signal PPG sur une période de temps comme entrée dans un réseau neuronal profond, DNN ; et

déterminer (106 ; 312), par l'intermédiaire du DNN, un modèle spécifique au sujet reliant le signal PPG du sujet à la fréquence cardiaque du sujet.

2. Procédé mis en oeuvre par ordinateur selon la revendication 1, comprenant en outre l'association du modèle spécifique à un sujet déterminé au sujet spécifique.

3. Procédé mis en oeuvre par ordinateur selon une quelconque des revendications 1 ou 2, dans lequel le DNN comprend une pile de couches de réseau neuronal.

4. Procédé mis en oeuvre par ordinateur selon la revendication 3, dans lequel la pile de couches de réseau neuronal comprend une ou plusieurs couches de réseaux neuronaux convolutifs, CNN (402 ; 404), qui effectuent une extraction automatique de caractéristiques de l'ensemble de données complet de la série temporelle du signal PPG.

5. Procédé mis en oeuvre par ordinateur selon la revendication 4, dans lequel la pile de couches de réseau neuronal comprend en outre une ou plusieurs couches de mémoire à long terme, LSTM (406 ; 408), qui capturent les propriétés temporelles du signal PPG.

6. Procédé mis en oeuvre par ordinateur selon une quelconque des revendications précédentes, dans lequel les données d'apprentissage spécifiques à un sujet reçues comprennent un ensemble de signaux PPG comprenant des signaux PPG acquis en relation avec différentes activités du sujet.

7. Procédé d'estimation de la fréquence cardiaque à partir d'un signal de photopléthysmographie, PPG, d'un sujet, ledit procédé comprenant de :

acquérir (202 ; 302, 304, 306, 310) des données d'apprentissage spécifiques à un sujet pour un apprentissage automatique, lesdites données d'apprentissage comprenant un signal PPG acquis par un dispositif portable provenant du sujet et un signal indiquant la fréquence cardiaque provenant du sujet, dans lequel le signal indiquant la fréquence cardiaque fournit une vérité de terrain des fréquences cardiaques du sujet pour associer une fréquence cardiaque à une période de temps du signal PPG ;

transférer (204) les données d'apprentissage spécifiques à un sujet à un processus d'apprentissage automatique ;

recevoir (206) un modèle appris par machine spécifique à un sujet issu du processus d'apprentissage automatique, le modèle définissant une relation d'un ensemble de données complet d'une série temporelle d'un signal PPG sur une période de temps pour une fréquence cardiaque du sujet ;

acquérir (208) un signal PPG par le dispositif portable pour estimer la fréquence cardiaque du sujet ; et

déterminer (210) une fréquence cardiaque du sujet sur la base du signal PPG acquis pour l'estimation de la fréquence cardiaque et du modèle spécifique au sujet.

8. Procédé selon la revendication 7, dans lequel les données d'apprentissage sont acquises pendant une période d'apprentissage initiale pour la génération du modèle appris par machine, et après que le modèle appris par machine spécifique au sujet a été généré, la détermination de la fréquence cardiaque du sujet sur la base du signal PPG acquis pour l'estimation de la fréquence cardiaque est activée.

9. Procédé selon la revendication 8, comprenant en outre la mise à jour du modèle appris par machine spécifique au sujet après que le modèle appris par machine spécifique au sujet a été initialement généré, ladite mise à jour comprenant pendant une période d'apprentissage à la mise à jour de :

acquérir des données d'apprentissage à la mise à jour spécifiques au sujet pour l'apprentissage automatique, lesdites données d'apprentissage à la mise à jour comprenant un signal PPG provenant du sujet et un signal indiquant la fréquence cardiaque provenant du sujet, dans lequel le signal indiquant la fréquence cardiaque fournit une vérité de terrain des fréquences cardiaques du sujet pour associer une fréquence cardiaque à une période de temps du signal PPG ;

transférer les données d'apprentissage à la mise à jour spécifiques au sujet à un processus d'apprentissage automatique ;

recevoir un modèle appris par machine spécifique à un sujet mis à jour provenant du processus d'apprentissage automatique, dans lequel le modèle mis à jour définit une relation d'un ensemble de données complet d'une série temporelle d'un signal PPG sur une période de temps à une fréquence cardiaque du sujet.

10. Procédé selon une quelconque des revendications 7 à 9, dans lequel l'acquisition (202 ; 302, 304, 306, 310) de données d'apprentissage spécifiques à un sujet pour l'apprentissage automatique comprend l'acquisition (302) d'un signal PPG provenant du sujet à l'aide d'un capteur PPG émettant une lumière verte vers une surface cutanée du sujet et détectant la lumière réfléchie depuis une surface cutanée du sujet.

11. Procédé selon une quelconque des revendications 7 à 10, dans lequel l'acquisition (202 ; 302, 304, 306, 310) de données d'apprentissage spécifiques à un sujet pour l'apprentissage automatique comprend l'acquisition (302) d'un signal PPG provenant du sujet à l'aide d'un capteur PPG et le prétraitement (304) du signal PPG.

12. Procédé selon la revendication 11, dans lequel le prétraitement (304) du signal PPG comprend le filtrage du signal PPG à l'aide d'un filtre passe-bande.

13. Procédé selon une quelconque des revendications 7 à 10, dans lequel l'acquisition (202 ; 302, 304, 306, 310) de données d'apprentissage spécifiques à un sujet pour l'apprentissage automatique comprend la réception (310) d'un signal d'électrocardiogramme, ECG, fournissant un signal indiquant la fréquence cardiaque du sujet.

14. Système (500) pour l'estimation de la fréquence cardiaque à partir d'un signal de photopléthysmographie, PPG, d'un sujet, ledit système (500) comprenant :

un capteur PPG (504) pour détecter un signal PPG provenant du sujet,

une unité de communication (506), qui est configurée pour communiquer avec un réseau neuronal pour l'apprentissage automatique, l'unité de communication (506) étant configurée pour acquérir à partir du capteur PPG (504) des données d'apprentissage spécifiques au sujet pour l'apprentissage automatique, lesdites données d'apprentissage comprenant le signal PPG provenant du sujet ; l'unité de communication (506) étant en outre configurée pour transférer les données d'apprentissage spécifiques au sujet au réseau neuronal, qui reçoit en outre un signal indiquant la fréquence cardiaque provenant du sujet, le signal indiquant la fréquence cardiaque fournissant une vérité de terrain des fréquences cardiaques du sujet pour associer une fréquence cardiaque à une période de temps du signal PPG, et l'unité de communication (506) étant en outre configurée pour recevoir du réseau neuronal un modèle appris par machine spécifique au sujet, le modèle définissant une relation entre un ensemble de données complet d'une série temporelle d'un signal PPG sur une période de temps et une fréquence cardiaque du sujet; et

un processeur (510), qui est configuré pour recevoir le modèle spécifique au sujet de l'unité de communication (506) et, après avoir reçu le modèle spécifique au sujet, recevoir le signal PPG du capteur PPG (504) pour une estimation de la fréquence cardiaque ; le processeur (510) étant en outre configuré pour déterminer une fréquence cardiaque du sujet sur la base du signal PPG reçu du capteur PPG (504) et le modèle spécifique au sujet ; et

un support ayant une forme adaptée pour être porté par un sujet et configuré pour transporter le capteur PPG (504), l'unité de communication (506) et le processeur (510).

15. Système selon la revendication 14, dans lequel le support (502) est configuré pour être porté sur le poignet d'un sujet.

Receive subject-specific training
data for machine learning — 102

Use associated pairs of heart rate
and complete dataset of time-series
of PPG signal as input to
deep neural network — 104

Determine subject-specific model
relating PPG signal to heart rate — 106

*Fig. 1*

Acquire subject-specific training
data for machine learning — 202

Transfer subject-specific training
data to machine learning process — 204

Receive subject-specific
machine-learned model — 206

Acquire PPG signal — 208

Determine heart rate based on PPG
signal and subject-specific model — 210

*Fig. 2*

EP 3 626 167 B1

Fig. 3

Fig.4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180249964 A1

**Non-patent literature cited in the description**

- **VASU JINDAL.** Integrating Mobile and Cloud for PPG Signal Selection to Monitor Heart Rate During Intensive Physical Exercise. *2016 IEEE/ACM International Conference on Mobile Software Engineering and Systems,* 16 May 2016, 36-37
- **MOYA-ALBOR et al.** Heart Rate Estimation using Hermite Transform Video Magnification and Deep Learning. *2018 40th Annual International Conference of the IEEE Engineering in Medicine and Biology Society (EMBC),* 18 July 2018, 2595-2598

- **ZHILIN ZHANG ; ZHOUYUE PI ; BENYUAN LIU.** TROIKA: A General Framework for Heart Rate Monitoring Using Wrist-Type Photoplethysmographic Signals During Intensive Physical Exercise. *IEEE Transaction on Biomedical Engineering,* February 2015, vol. 62 (2), 522-531